# EUROPÄISCHE PATENTANMELDUNG

(11) **EP 0 885 628 A2**
(43) Veröffentlichungstag der Anmeldung: **23.12.1998**
(21) Anmeldenummer: 98110769.1
(22) Anmeldetag: 12.06.1998
(51) Int. Cl.: A61N 1/40, A61N 2/02

(54) **Elektronisches Bioresonanzgerät**

(30) Priorität: 18.06.1997 CH 1480/97
(71) Anmelder: Coufal Elektronik AG, 9427 Wolfhalden (CH)
(72) Erfinder: Coufal, Hanspeter, 9427 Wolfhalden (CH)
(74) Vertreter: Römpler, Aldo

(57) **Zusammenfassung**

Das vom Körper des Patienten stammende Eingangs-Signal wird vorzugsweise über eine elektrisch leitenden Matte (7) zum Grundgerät (1) geleitet. In diesem wird das empfangene elektrische bzw. elektromagnetische Signal mittels eines Verstärkers (3) verstärkt und über eine als Antenne dienende Sendespule (4) abgestrahlt. Das Ausgangs-Signal (5) bzw. die Rücklauf-Energie erreicht den Patienten somit am ganzen Körper. Dadurch werden einerseits Herzbeschwerden vermieden und andererseits eine bessere therapeutische Wirkung erzielt.

## Beschreibung

Die vorliegende Erfindung betrifft ein Elektronisches Bioresonanz-Gerät.

Ein Bioresonanz-Gerät ist insbesondere dazu bestimmt, die elektrischen Signale eines menschlichen oder tierischen Körpers aufzunehmen und an diesen zurückzugeben. Die Bioresonanz-Therapie ist seit Jahrzehnten bekannt. Sie beruht darauf, dass in jeder Zelle neben chemischen auch elektrische Aspekte eine Rolle spielen, die sich in schwachen elektrischen bzw. elektromagnetischen Strömen äussern. Diese Ströme dienen auch der Übertragung von Körperinformationen. Als Folge hiervon weist jedes Lebewesen ein elektromagnetisches Feld auf. Gemeinhin spricht man auch von körpereigenen Schwingungen. Bei der Bioresonanz-Therapie wird davon ausgegangen, dass gesundheitliche oder verletzungsbedingte Störungen gleichfalls zu einer Störung bzw. Veränderung des elektromagnetischen Feldes führen. Durch die Veränderung der Schwingungen breitet sich die Information der Störung des Gesundheitszustandes im ganzen Körper aus. Durch Aufnahme dieser Schwingungen bzw. dieser elektromagnetischen Signale und durch deren Rückgabe an den Körper wird, nach dem physikalischen Prinzip der Invers-Schwingung, eine Löschung erreicht. Anders ausgedrückt, die durch die Veränderung des elektromagnetischen Feldes weitergeleitete Information der Störung des Gesundheitszustandes wird unterdrückt. Wie sich aus der langjährigen Erfahrung mit der Bioresonanz-Therapie ergeben hat, kann damit eine Besserung des Wohlbefindens des Patienten und eine Unterstützung der natürlichen Hellungsprozesse erreicht werden. Der besondere Vorteil der Bioresonanz-Therapie liegt darin, dass die positive Wirkung ohne chemische Mittel und somit ohne die damit einher gehenden Nebenwirkungen erzielt wird. Man beachte zudem, dass im Gegensatz zur Elektroakupunktur, dem Patienten keine Fremdsignale zugeleitet werden.

Die bisher bekannten Bioresonanz-Geräte arbeiten mit zwei Elektroden, wobei der Patient je eine in eine Hand zu nehmen hat. Durch die erste Elektrode werden die elektromagnetischen Signale vom Körper aufgenommen und durch die zweite Elektrode, nach einer entsprechenden Verstärkung, wieder an diesen abgegeben. Die beiden Elektroden sind mittels Kabel mit einem Grundgerät verbunden. Wenn nun das dem Körper zugeleitete, verstärkte Signal diesen über eine Hand erreicht, so wird es durch den Arm zwangsläufig direkt in den Brustbereich und damit gezielt zum Herzen des Patienten geleitet. Es kann also eine unerwünschte elektromagnetische Reizung des Herzens bewirkt werden. Dies ist insbesondere bei Patienten mit Herzbeschwerden sehr ungünstig.

Die vorliegende Erfindung stellt sich daher die Aufgabe, ein Elektronisches Bioresonanz-Gerät zu schaffen, das einerseits den vorgenannten Nachteil nicht aufweist und andererseits über verbesserte Einsatzmöglichkeiten verfügt.

Das erfindungsgemässe Gerät entspricht den kennzeichnenden Merkmalen des Patentanspruchs 1. Weitere vorteilhafte Ausbildungen gehen aus den abhängigen Patentansprüchen hervor.

Bisher wurde grundsätzlich davon ausgegangen, dass die hier auftretenden elektromagnetischen Signale zumindest teilweise nicht abstrahlfähig bzw. nicht antennengängig sind. Wie sich bei Patientenversuchen gezeigt hat, ist diese in der Fachwelt geltende Annahme überraschenderweise nicht ganz zutreffend. So ist es zwar zum Empfang der vom Körper kommenden Signale besser mit einer Elektrode oder mit einer ähnlichen Einrichtung zu arbeiten, die allerdings ebenfalls nicht unbedingt im direkten Hautkontakt mit dem Patienten stehen muss. Das Zurückgeben der verstärkten Signale kann aber durchaus über eine Antenne bzw. Sendespule erfolgen, sofern beachtet wird, dass die Sendereichweite auf 1 - 2 m beschränkt ist. Durch diese in bezug auf Empfang und Aussendung der Signale geänderten Vorgaben ergeben sich deutlich verbesserte Anwendungs- bzw. Therapiemöglichkeiten, die mittels des erfindungsgemässen, elektronischen Bioresonanzgerätes voll ausgeschöpft werden können.

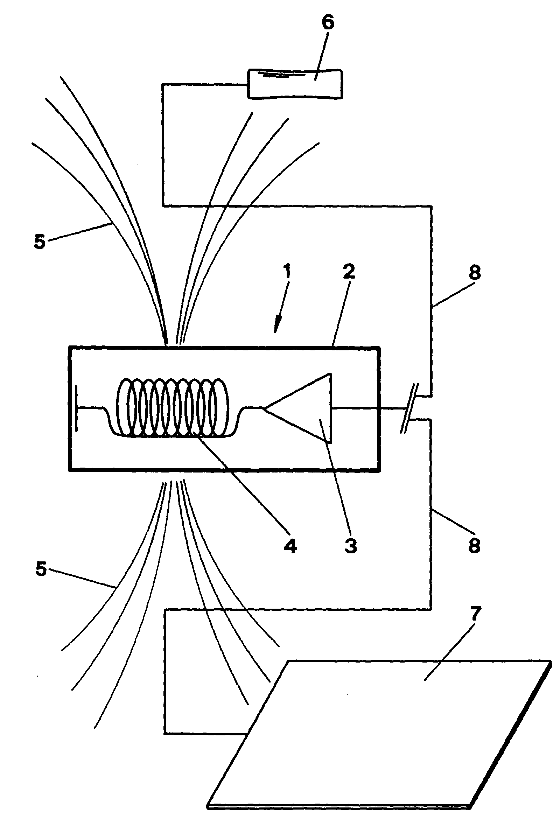

Nachfolgend wird ein bevorzugtes Ausführungsbeispiel des Erfindungsgegenstandes anhand der Zeichnung näher beschrieben.

Das Grundgerät 1 ist in einem Gehäuse 2 untergebracht, wobei einfachheitshalber nur die erfindungswesentlichen Bauteile schematisch dargestellt sind. Das vom Körper des Patienten ankommende Eingangs-Signal wird im Grundgerät 1 durch einen Verstärker 3 verstärkt und über eine als Antenne wirkende Sendespule 4 als Ausgangs-Signal 5 ausgesandt. Das Eingangs-Signal gelangt entweder über eine übliche Hand-Elektrode 6 oder aber über eine elektrisch leitende Platte oder Matte 7 vom Patienten zum Grundgerät 1. Die Hand-Elektrode 6 kann auch direkt am Grundgerät 1 angeordnet sein, d.h. ohne Verbindungskabel 8. Dieses Verbindungskabel 8 sollte im übrigen nicht länger als 1 - 2 m sein, da ansonsten das vom Körper des Patienten stammende Eingangs-Signal vom Grundgerät 1 nicht zuverlässig empfangen werden kann. Im vorliegenden Fall entspricht diese Entfernung praktischerweise zugleich der durch Versuche ermittelten, therapeutisch wirksamen Reichweite des von der Sendespule 4 abgestrahlten Ausgangs-Signals 5.

Insbesondere durch die Verwendung einer Matte 7 kann das vom Körper des Patienten stammende elektrische bzw. elektromagnetische Signal kapazitiv nahe am Körper aufgenommen werden. Dies bietet eine Reihe von Vorteilen. So kann die Matte 7 beispielsweise auf die Sitz- oder Lehnfläche eines Stuhles gelegt werden, wobei das Signal grossflächig vom Körper empfangen und als Eingangs-Signal zum Grundgerät 1 geleitet werden kann, ohne dass sich der Patient zu entkleiden braucht. Die Matte 7 kann zudem einem liegenden Patienten beispielsweise auf die Brust gelegt werden. Das Eingangs-Signal kann somit von einer entsprechend grossen Körperfläche beliebig am Rumpf oder an einem der Glieder des Patienten aufgenommen werden. Die Matte 7 ermöglicht darüber hinaus die Anwendung der Bioresonanz-Therapie auch bei Tieren, wo eine herkömmliche Hand-Elektrode 6 selbsverständlich unbrauchbar wäre.

Das über die Antenne bzw. Sendespule 4 abgestrahlte Ausgangs-Signal 5 erreicht den Körper des Patienten ebenfalls grossflächig, indem dieses nicht, wie bei einer herkömmlichen Hand-Elektrode, nur über eine einzige Hand empfangen wird. Durch die erfindungsgemässe Ausbildung des Bioresonanzgerätes wird das Ausgangs-Signal 5, bzw. die Rücklaufenergie, magnetisch in den ganzen Körper des Patienten verteilt. Dadurch ist die Belastung des Herzens erheblich vermindert, so dass hier keine schädlichen Auswirkungen zu gewärtigen sind. Dieses Gerät ist selbst bei Patienten mit Herzschrittmacher gefahrlos einsetzbar. Es versteht sich ausserdem von selbst, dass durch die Verteilung der Energie des Ausgangs-Signals 5 auf den gesamten Körper eine bessere therapeutische Wirkung erzielbar ist. In jedem Fall ergibt sich, zumal bei Verwendung der Matte 7, eine optimal einfache und entspannende Behandlung von Mensch und Tier.

Im Grundgerät 1 sind neben dem Verstärker und der Sendespule 4 selbstverständlich noch weitere Bauteile vorzusehen, wie insbesondere eine Stromversorgung und eine Steuerung zur Regelung der Betriebsabläufe sowie allenfalls Bedienungselemente, Betriebsanzeigen und Ein- bzw. Ausgangsbuchsen. Sinnvoll ist auch die Messung des Eingangs-Signals, z.B. über einen Eingangs-Widerstand, so dass das Ausgangs-Signal durch die Steuerung und den Verstärker entsprechend bearbeitet werden kann. Ebenso ist neben einer Hand-Elektrode 6 oder einer Matte 7 zusätzliches Zubehör denkbar. Es liegt indessen Im Rahmen der Erfindung das Bioresonanzgerät anders als vorgehend beschrieben auszubilden.

## Patentansprüche

1. Elektronisches Bioresonanzgerät, gekennzeichnet durch eine Einrichtung (6, 7) zum Empfang eines elektrischen bzw. elektromagnetischen Feldes als Eingangs-Signal, wobei für das Abstrahlen des Ausgangs-Signals (5) eine Sendevorrichtung (4) vorgesehen ist.

2. Elektronisches Bioresonanzgerät nach Anspruch 1, dadurch gekennzeichnet, dass die zum Abstrahlen des an einen menschlichen oder tierischen Patienten zurückzugebenden Ausgangs-Signals (5) des von der Empfangs-Einrichtung (6, 7) als Eingangs-Signal aufgenommenen, körpereigenen elektrischen bzw. elektromagnetischen Feldes dienende Sendevorrichtung eine Antenne oder Sendespule (4) aufweist.

3. Elektronisches Bioresonanzgerät nach Anspruch 1 oder 2, dadurch gekennzeichnet, dass ein Verstärker (3) vorgesehen ist, mit dem Zweck das Eingangs-Signal vor dessen Abstrahlen als Ausgangs-Signal (5) zu verstärken.

4. Elektronisches Bioresonanzgerät nach einem der Ansprüche 1 - 3, dadurch gekennzeichnet, dass zum Empfang des Eingangs-Signals mindestens eine flächige, starre oder biegsame, elektrisch leitende Empfangseinrichtung (7) vorgesehen ist.

5. Elektronisches Bioresonanzgerät nach Anspruch 4, dadurch gekennzeichnet, dass die Empfangseinrichtung mindestens eine elektrisch leitende Matte (7) aufweist.

6. Elektronisches Bioresonanzgerät nach einem der Ansprüche 1 - 5, gekennzeichnet durch eine die Betriebsabläufe regelnde Steuerung.

7. Elektronisches Bioresonanzgerät nach einem der Ansprüche 1 - 6, gekennzeichnet durch eine mit der Steuerung und dem Verstärker (3) verbundenen Mess-Einrichtung, beispielsweise einem Eingangs-Widerstand, zur Messung des Eingangs-Signals.
